# EUROPEAN PATENT SPECIFICATION

(11) **EP 2 790 750 B1**
(45) Date of publication and mention of the grant of the patent: **07.11.2018**
(21) Application number: 12857884.6
(22) Date of filing: 14.12.2012
(51) Int. Cl.: A61M 5/00, A61M 39/10, A61J 15/00

(54) **CHANNEL SEPARATION DEVICE AND RELATED METHOD THEREOF**
KANALTRENNVORRICHTUNG UND ENTSPRECHENDES VERFAHREN
DISPOSITIF DE SÉPARATION DE CANAL ET PROCÉDÉ ASSOCIÉ

(30) Priority: 16.12.2011 US 201161576775 P; 17.01.2012 US 201261587580 P
(43) Date of publication of application: 22.10.2014
(73) Proprietor: University of Virginia Patent Foundation, Charlottesville, VA 22902 (US)
(72) Inventor: ROSENBERGER, Laura, H., Charlottesville, Virginia 22902 (US); SAWYER, Robert, G., Charlottesville, Virginia 22901 (US); WATSON, Christopher, M., Columbia, South Carolina 29169 (US)
(74) Representative: Appelt, Christian W.
(86) International application number: PCT/US2012/069793
(87) International publication number: WO 2013/090748

(56) References cited:
- WO-A1-2005/004974
- US-A1- 2004 111 056
- US-A1- 2005 033 267
- US-A1- 2008 191 466
- US-A1- 2008 191 466
- US-A1- 2009 221 964
- US-A1- 2010 076 383
- US-A1- 2011 112 482
- US-B1- 7 730 847
- US-B1- 7 766 877
- ROSENBERGER, L ET AL.: 'Accidental dislodgement of the Percutaneous Endoscoptic Gastrostomy (PEG): a novel SafetyBreak device for global prevention.' 14 April 2011, XP055156680 Retrieved from the Internet: <URL:http://www.virginia.edu/inauguration/p osters/6.214.TranslationaLRosenberger.pdf> [retrieved on 2013-02-20]

## Description

### FIELD OF THE INVENTION

The present invention relates generally to the field of medical devices. More specifically, the invention is in the subfield of tubing, channels, drains, catheters, and ports connected to a subject patient.

### BACKGROUND OF THE INVENTION

The percutaneous endoscopic gastrostomy, or the PEG, as it is now most commonly known, was first introduced in 1980 by Gauderer, Ponsky, and Izant. Currently, more than 215,000 PEGs are placed annually (See Gauderer et al., "Percutaneous Endoscopic Gastrostomy-20 Years Later: A Historical Perspective", Journal of Pediatric Surgery, 2001, vol. 36(1), pp. 217-219). Due to the design of the PEG, a major complication is "premature removal" or accidental dislodgement, from which significant morbidity and mortality may occur, as for example shown in Schapiro et al., "Complications of percutaneous endoscopic gastrostomy", Gastrointest Endosc Clin N Am, 1996, vol. 6, pp. 409-422. As shown in Chowdhury et al., "Complications and outcome of percutaneous endoscopic gastrostomy indifferent patient groups", Journal of Gastroenterology and Hepatology, 1996; col. 11, pp. 835-839, secondary complications occur with premature removal, as the gastrocutaneous fistula tract has not fully matured, allowing the stomach to separate from the anterior abdominal wall and the open gastrostomy to leak gastric contents. Accidental dislodgement rates are well published and have been reported up to 12.8% when followed longitudinally for the lifetime of the originally placed PEG.

While early dislodgements can be clinically devastating, late dislodgements after gastrocutanous fistula maturation are less detrimental, however, they may require expensive emergency department visits, surgical consultations, replacement tubes, and radiographic confirmation of position as shown in Rosenberger et al., "Late accidental dislodgement of the percutaneous endoscopic gastrostomy: an underestimated burden on patients and the healthcare system", Surgical Endoscopy, 2011, vol. 25, pp. 3307-3311.

The mechanism that causes disruption of the PEG, may also cause disruption of any channel that is positioned inside a subject. Catheters, drains, and various other tubes are all subject to disruption when tractive force is applied to the portion of those tubes that are external to the subject.

Therefore, there is need in the art for a device which reduces the disruption of channels placed inside a subject.

US 2011/0112482 discloses a PEG tube connector releasable between two tubing segments to avoid accidental removal of a distal portion of a PEG tube from a patient's abdomen when the tube is pulled on.

US 2008/0191466 discloses a fluid connector having a high separating resistance axially and a low separating resistance transverse to the axial axis.

WO 2005/004974 discloses a coupling device for coupling a patient-side medical line to an equipment-side medical line.

### SUMMARY OF THE INVENTION

The invention is defined by the subject matter of independent claim 1. Advantageous embodiments of the invention are subject to the dependent claims.

An aspect of an embodiment of the present invention provides an assembly device for use in a medical environment to be used with a first channel and a second channel, wherein the first channel has an internal portion located at an intended position inside a subject. The device may comprise: a first connector comprising a first interface member and a channel communication section, at least a portion of said first channel communication section is provide inside said first channel; a second connector comprising a second interface member and a channel communication section, at least a portion of said second channel communication section is provided inside said second channel; said first interface member of said first connector and said second interface member of said second connector configured to join together to form a coupling configured to allow fluid to flow between the first channel and the second channel, said coupling having a predetermined decoupling force; wherein said coupling is joined to the first channel and the second channel at said communication section of said first connector and said communication section of said second connector; and wherein said predetermined decoupling force is the force required to separate said first connector and the second connector from one another to allow the internal portion to maintain its intended position in the subject.
An example useful for understanding the present invention provides an assembly device for use in a medical environment to be used with a first channel and a second channel, wherein the first channel has an internal portion located at an intended position inside a subject. The device may comprise: a first connector; a second connector; said first connector and said second connector configured to join together to form a coupling configured to allow fluid to flow between the first channel and the second channel, said coupling having a decoupling force; wherein said coupling is joined between the first channel and the second channel; and wherein said decoupling force is the force required to separate said first connector and the second connector from one another to allow the internal portion to maintain its intended position in the subject.

An aspect of an embodiment of the present invention provides a system for use in a medical environment. The system may comprise: a first channel; a second channel, wherein the first channel has an internal portion located at an intended position inside a subject; and an assembly device. The assembly device may comprise: a first connector comprising a first interface member and a channel communication section, at least a portion of said first channel communication section is provided inside said first channel; a second connector comprising a second interface member and a channel communication section, at least a portion of said second channel communication section is provided inside said second channel; said first interface member of said first connector and said second interface member of said second connector configured to join together to form a coupling configured to allow fluid to flow between said first channel and said second channel, said coupling having a predetermined decoupling force; wherein said coupling is joined to said first channel and said second channel at said communication section of said first connector and said communication section of said second connector; and wherein said predetermined decoupling force is the force required to separate said first connector and the second connector from one another to allow the internal portion to maintain its intended position in the subject.

An example useful for understanding the present invention provides a system for use in a medical environment, wherein said system comprises a first channel; a second channel, wherein the first channel has an internal portion located at an intended position inside a subject; and an assembly device. The assembly device may comprise: a first connector; a second connector; said first connector and said second connector configured to join together to form a coupling configured to allow fluid to flow between said first channel and said second channel, said coupling having a decoupling force; wherein said coupling is joined between said first channel and said second channel; and wherein said decoupling force is the force required to separate said first connector and the second connector from one another to allow the internal portion to maintain its intended position in the subject.
An example useful for understanding the present invention provides a method used in a medical environment to be used with a first channel and a second channel, wherein said first channel has an internal portion located at an intended position inside the subject. The method may comprise: providing a first connector; providing a second connector; joining said first connector and said second connector to form a coupling to allow fluid to flow between the first channel and the second channel, said coupling having a decoupling force; wherein said coupling is joined to the first channel and the second channel; and wherein said decoupling force is the force required to separate said first connector and said second connector from one another to allow the internal portion to maintain its intended position.

An example useful for understanding the present invention provides a method used in a medical environment. The method may comprise: providing a first channel; providing a second channel, wherein said first channel has an internal portion located at an intended position inside the subject; providing a first connector; providing a second connector; joining said first connector and said second connector to form a coupling to allow fluid to flow between said first channel and said second channel, said coupling having a decoupling force; wherein said coupling is joined to said first channel and said second channel; and wherein said decoupling force is the force required to separate said first connector and said second connector from one another to allow the internal portion to maintain its intended position.

These and other advantages and features of the invention disclosed herein, will be made more apparent from the description, drawings and claims that follow.

### DESCRIPTION OF THE DRAWINGS

The accompanying drawings illustrate several aspects and embodiments of the present invention and, together with the description herein, serve to explain the principles of the invention. The drawings are provided only for the purpose of illustrating select embodiments of the invention and are not to be construed as limiting the invention.
**Figure 1** provides a schematic illustration of the assembly device in use with a subject.
**Figures 2(A)** **and** **2(B)** provide schematic illustrations of the assembly device in use with a subject. **Figures 2(A)** **and** **2(B)** also illustrate an embodiment of the first interface member on the first connector and an embodiment of the second interface member on the second connector. **Figure 2(B)** further illustrate an embodiment of the protrusions of the first interface member and the protrusion retention recesses of the second interface member.
**Figures 3(A)****-(D)** provide sectional views of several specific embodiments of the first connector having different embodiments of the channel communication section. It should be appreciated that these embodiments of the channel communication section are equally applicable to the second connector.
**Figure 4** provides a sectional view of an embodiment of the assembly device in use with a subject.
**Figure 5(A)** provides an elevation view of an embodiment of the first connector. **Figure 5(B)** provides a sectional view designated as A-A for the embodiment shown in **Figure 5(A)****.**
**Figure 5(C)** provides an enlarged partial view designated as Detail B of the sectional view shown in **Figure 5(B)****.**
**Figure 6(A)** provides an elevation view of an embodiment of the second connector.
**Figure 6(B)** provides a sectional view designated as B-B for the embodiment shown in **Figure 6(A)****.**
**Figure 6(C)** provides an enlarged partial view designated as Detail A of the sectional view shown in **Figure 6(B)****.**
**Figure 7(A)** provides an elevation view of an embodiment of the coupling formed by the joining of the first connector shown in **Figure 5(A)** and the second connector show in **Figure 6(A)****.**
**Figure 7(B)** provides a sectional view designated as C-C for the embodiment shown in **Figure 7(A)****.**
**Figure 7(C)** provides an enlarged partial view designated as Detail D of the sectional view shown in **Figure 7(B)****.**
**Figures 8(A)-8(B)** provide a perspective view of an embodiment of the coupling in a disconnected position and connected position, respectively.
**Figure 9(A)** provides an illustration of an elevation schematic view of a PEG tube. **Figures 9(B)****-(C)** provides an elevation schematic view of an embodiment of the coupling in a disconnected position and connected position, respectively.
**Figure 10** graphically illustrates the force (kg) of external traction for removal of PEG tubes through abdominal wall versus the thickness (cm) of the abdominal wall.
**Figure 11** graphically illustrates the force of external traction (kg) versus "age" (days) of the PEG tube.
**Figure 12** graphically illustrates the Standard (Gaussian) distribution with mean (µ) and standard deviations (σ) used for determining the preferred break force.
**Figure 13** graphically illustrates the Kaplan-Meier analysis for current prospective cohort (Group 2) versus historic cohort (Group 1).

### DETAILED DESCRIPTION OF EXEMPLARY EMBODIMENTS OF THE INVENTION

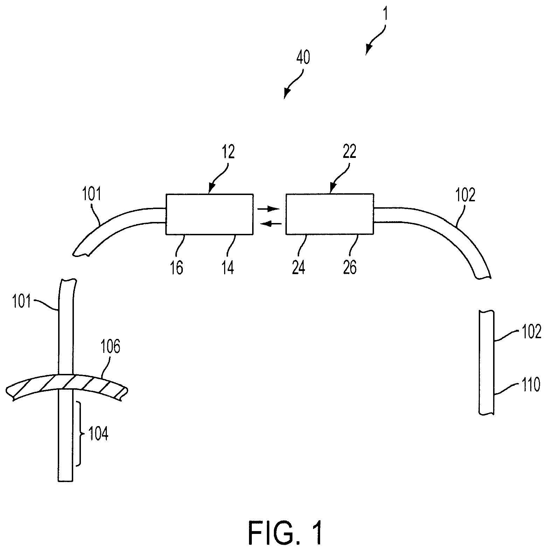

**Figure 1****,** provides a schematic illustration of the assembly device **1** in use with a subject. The device **1** is used in a medical environment and is to be used with a channel such as the first channel **101** and second channel **102** shown, and wherein the channel has an internal portion **104** that is located at an intended position inside a subject **106.** The device **1** includes a first connector **12** having a first interface member **14** and a channel communication section **16** and a second connector **22** having a second interface member **24** and a channel communication section **26.** The first interface member **14** of the first connector **12** and the second interface member **24** of the second connector **22** are configured to join together in the general directions of the arrows, to form a coupling **40** that is configured to allow fluid (or other material or gas) to flow between the first channel **101** and the second channel **102.** The coupling **40** exhibits a desired or predetermined decoupling force. In an embodiment, the coupling **40** is joined to the first channel **101** and the second channel **102** at a communication section **16** of the first connector **12** and a communication section **26** of the second connector **22** in such manner so as to exhibit the decoupling force whereby the decoupling force is the force required to separate the first connector **12** and the second connector **22** from one another to allow the internal portion **104** to maintain its intended position in the subject **106.** Moreover, the separated first connector **12** and second connector **22** is able to remain joined to their respective channels.

It should be appreciated that the first connector and second connector may be interchanged with one another respective to the first channel and second channel.

Still referring to the assembly device **1** of **Figure 1****,** it should be appreciated that the first connector **12** and the second connector **22** forming the coupling **40** may be achieved by a number of designs and approaches. Some examples include, but are not limited to, male to female friction connectors, magnetic connectors, connectors that use adhesive, friction connection, any ridge/valley connection, snap/click connection, any O-ring connection, and screw/twist type mechanisms.

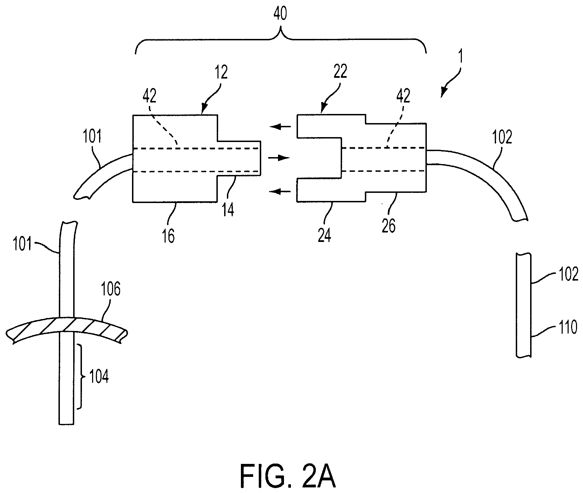

For instance, referring to the device of assembly of **Figure 2(A)****,** which provides schematic illustrations of the assembly device **1** in use with a subject **106,** the first connector **12** may include a male connector whereby the first interface member **14** provides an insert section. Similarly, the second connector **22** may include a female connector whereby the second interface member **24** provides a receiving section. As a result, the receiving section of the female connector may receive the insert section of the male connector so as to join together the two connectors to form the coupling **40.**

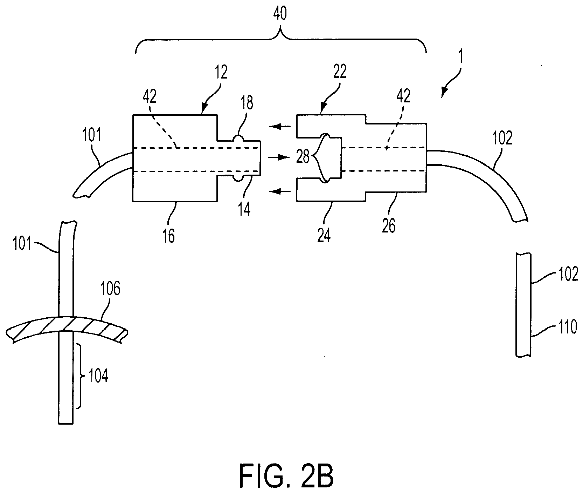

Referring to **Figure 1** and **Figures 2(A)** and **2(B),** the channel (e.g., first channel **101** and second channel **102**) may include at least one of the following: a tube, a conduit, a port or any combination thereof. By way of example, in an embodiment the tube or the like may be connected to the channel communication section **16** of the first coupling **12,** and that tube or the like may be connected to a port (not shown) that is within and/or adjacent to the internal portion **104** inside the subject **106.** As such, the tube and the port would be connected to comprise the first channel **101.** Next, in an embodiment the distal end **110** of the second channel **102** may be attached to a biomedical device such as a feeding apparatus or drainage collection vessel; however, it should be noted that the invention is not limiting in this regard and the terminus of the second channel is determined by any number of medical uses.

Again referring to **Figure 1** and **Figures 2(A)** and **2(B),** in an embodiment the first channel **101** and second channel **102** may comprise a PEG tube, such that once fully assembled, the coupling **40** is located between the subject **106** and the distal end **110** of the second channel **102.**

Still referring to **Figure 1** and **Figures 2(A)** and **2(B),** the coupling **40** may have a decoupling force between 0.5 kilogram-force and 2.5 kilograms-force. The coupling **40** will separate when an appropriately oriented axial force is applied to the first connector **12** and second connector **22.** The force required to decouple the coupling **40** is designed to be less than the force required to disrupt the position of the internal portion **104** of the first channel **101** that is inside the subject **106.** In this way, the second channel **102** may be pulled either inadvertently or purposefully and the coupling **40** will separate before the force required to disrupt the portion of the first channel **101** inside the subject **106** is reached.

Still referring to **Figure 1** and **Figures 2(A)** and **2(B),** the coupling **40** may have a decoupling force between 1.1 kilograms-force and 1.3 kilograms-force. The coupling **40** will separate when an appropriately oriented axial force is applied to the first connector **12** and second connector **24.** The force required to decouple the coupling **40** is designed to be less than the force required to disrupt the position of the internal portion **104** of the first channel **101** that is inside the subject **106.** In this way, the second channel **102** may be pulled either inadvertently or purposefully and the coupling **40** will separate before the force required to disrupt the portion of the first channel **101** inside the subject **106** is reached.

It should be appreciated that the decoupling force may be increased or decreased so as to comply with operative, structural and anatomical demands associated with design and practice of the various embodiments disclosed herein.

Referring now to **Figure 2(B)****,** providing schematic illustrations of the assembly device **1** in use with a subject **106,** illustrates an embodiment including the protrusions **18** of the first interface member **14** and the protrusion retention recesses **28** of the second interface member **24.** When the first interface member **14** is inserted into the second interface member **24,** the circumferentially oriented protrusions **18** on the first interface member **14** causes the second interface member **24** to deform radially outward. This outward deformation continues to increase as the first interface member **14** is inserted farther into the second interface member **24.** When the first interface member **14** reaches its intended position inside the second connector **22,** the circumferentially oriented protrusions **18** on the first interface member **14** come to rest inside the protrusion retention recess or recesses **28** of the second interface member **24.** This causes the second interface member **24** to return to its original, non-deformed, shape and the coupling **40** is created. When the coupling **40** is created, a leak-inhibiting seal is created at the faying surfaces of the connection. The faying surfaces are all of the surfaces where the first connector **12** and the second connector **22** are in contact. It is well understood in the art that the dimensions, tolerances, and materials of the first and second connector affect the leak-inhibiting properties of the coupling **40.**

Still referring to **Figure 2(B)****,** the circumferentially oriented protrusions **18** may take many forms, including, but in no way limited to, a single continuous circumferential rib, a segmented circumferential rib, or multiple combinations of either form. The protrusion retention recess or recesses **28** are designed to accept the protrusions on the male connector insert section when the two connectors are joined. In this way, if there are multiple continuous circumferential ribs on the first interface member **14,** there shall be multiple protrusion retention recesses on the second interface member **24.** It should be appreciated that a single protrusion retention recess **28** may be adequate to accept a segmented circumferential rib, in this way, the radial orientation of the first connector **12** and the second connector **24** may be of no consequence when the connector are joined together to form a coupling. It is appreciated that a continuous circumferential rib may provide better leak-inhibiting characteristics than a segmented circumferential rib.

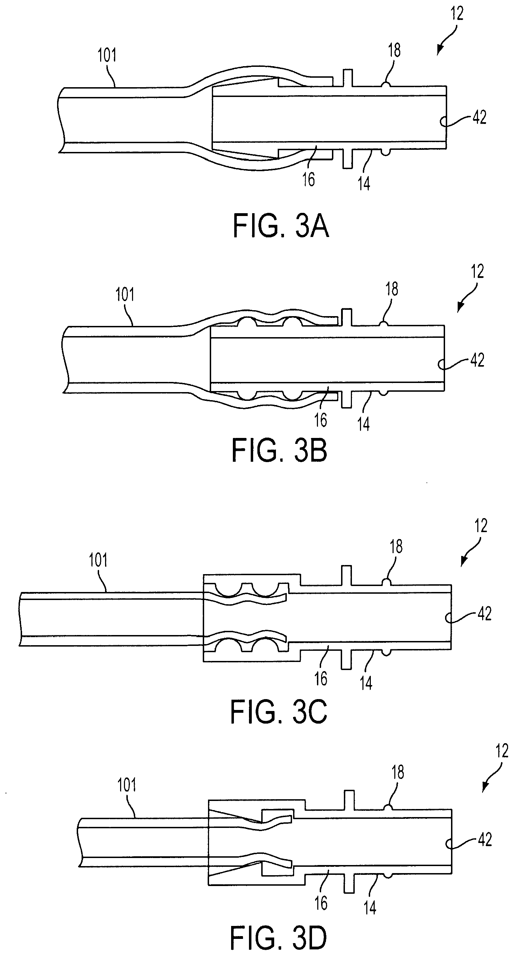

Referring now to **Figures 3(A)****-(D),** which provide sectional views of several specific embodiments of the channel communication section **16** typical on the first connector **12.** The channel communication section **16** of the first connector **12** (and channel communication section **26** the second connector **22** although not shown in **Figure 3****)** can be arranged in various forms known in the art. The first channel **101** (or second channel **102,** although not shown in **Figure 3**) may take the form of a tube, conduit, port, or other similar apparatus. As shown in **Figures 1** and **2****,** in an embodiment, a first tube **101** will be attached to the first connector **12** and a second tube **102** will be attached to the second connector **22** at their respective channel communication sections, **16** and **26**). **Figures 3(A)****-(D)** illustrate various, but not exhaustive, means to attach a first channel **101** to the channel communication section **16** of the first connector **12.** It should be appreciated that the same channel communication configurations shown on the first connector **12** also will apply equally to the second connector **22.** In **Figures 3(A)** and **3(B)****,** a first channel **101** is extended radially outward as the connector **12** is inserted into the first channel **101.** The first channel **101** expands to accept the connector **12** and is held in place by a friction force. This friction force is enhanced by an appendage **17,** such as the barb-like extension shown in **Figure 3(A)** and the two circumferentially oriented ribs extensions shown in **Figure 3(B). Figures 3(C)** and **3(D)** show configurations of the channel insert section **16** where the first channel **101** is compressed radially inward as the connector **12** is pushed over the first channel **101.** In Figure **3(C)****,** the channel **101** is held in place by a friction force generated by the appendage **17,** such as circumferentially oriented ribs on the inside of the connector **12.** In **Figure 3(D)****,** the channel **101** is held in place by a friction force generated by the appendage **17,** such as the barb-like extension on the inside of the connector **12.** Various combinations or means for connecting the channel **101** with the connector **12** shown in **Figures 3(A)-****(D)** may be used to obtain the desired strength or means of connection and it is understood that there are many other means commonly used in the art to attach a channel, tube, conduit, or port to a connector (**12** or **22**). Some other examples may include, but are not limited to, other type of male to female friction connectors, magnetic connectors, connectors that use adhesive, friction connection, any ridge/valley connection, snap/click connection, any O-ring connection, and screw/twist type mechanisms.

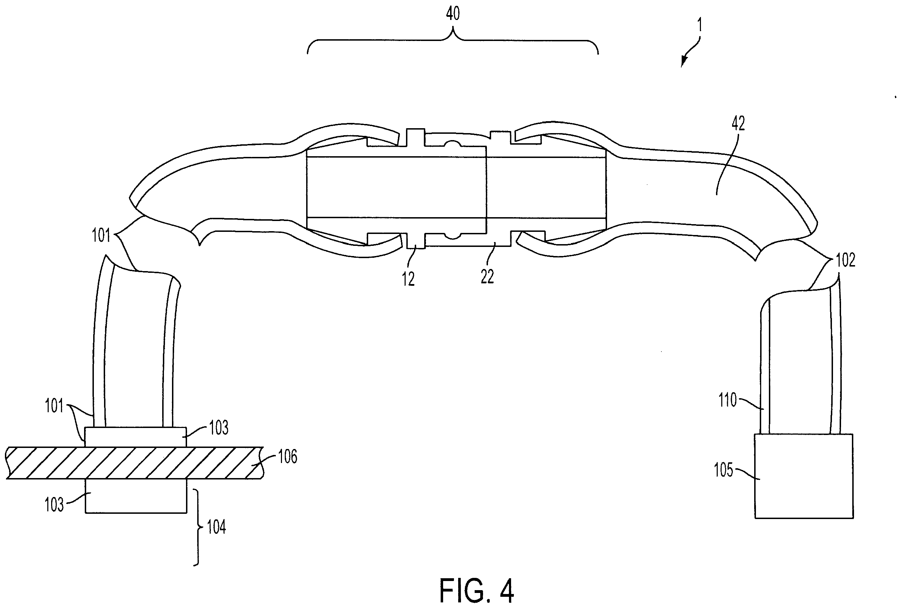

Referring now to **Figure 4, Figure 4** provides schematic illustrations of the assembly device **1** in use with a subject **106.** It is shown that the first channel **101** may be a tube that is connected to the channel communication section **16** of the first coupling **12,** and that tube may be connected to or be integral with a port **103** that has an internal portion **104** inside the subject **106.** Both the tube **101** and the port **103** connected together are may constitute portions of the first channel **101.** The distal end **110** of the second channel **102** is shown to be attached to a device or instrument **105** (biomedical device), such as a feeding apparatus or drainage collection vessel; however, it should be noted that the invention is not limiting in this regard and the terminus of the second channel **102** may be determined by any number of medical uses.

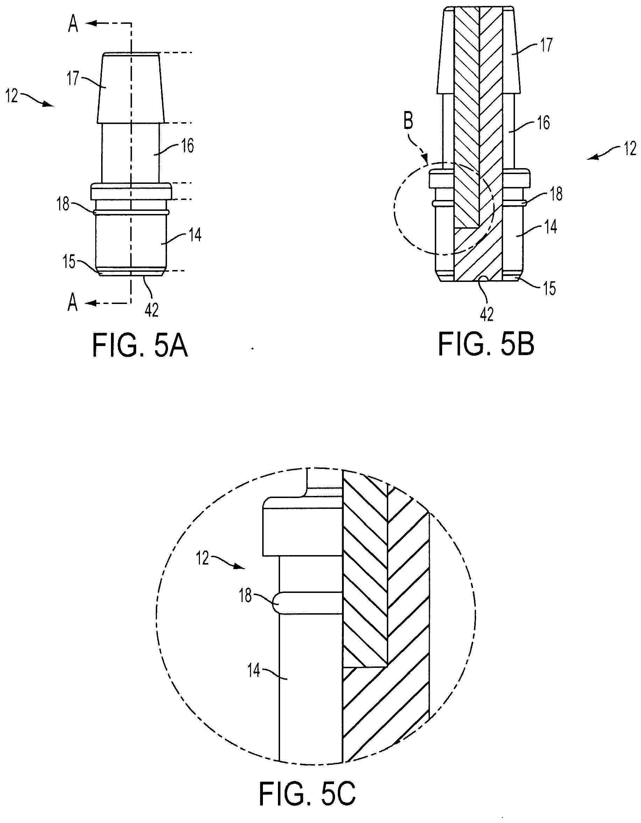

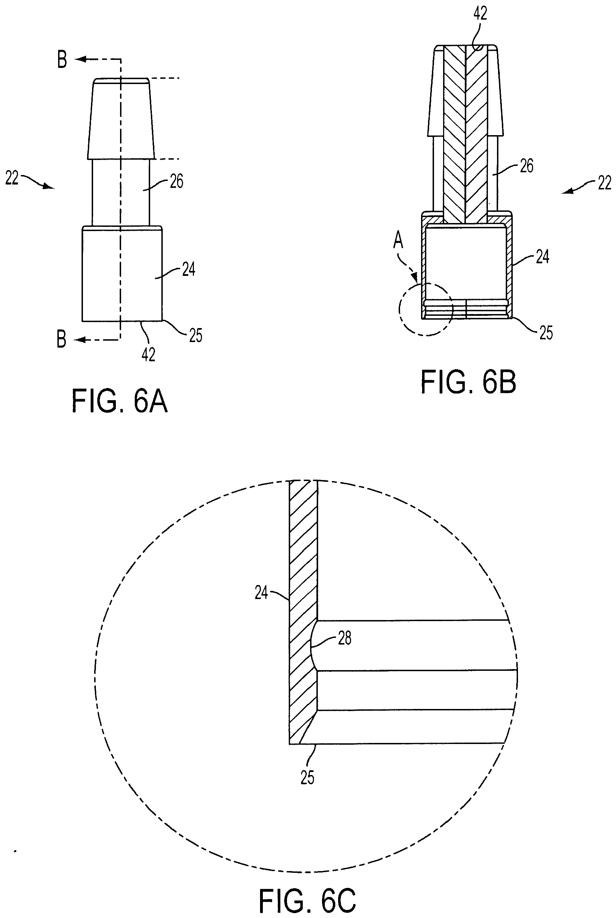

Referring now to **Figures 5(A)****-(C),** which provides assorted views of an embodiment of a first connector **12,** **Figure 5(A)** shows an elevated schematic of a first connector **12.** In this instance, the first interface member **14** is a male type connector with a first edge **15.** The first edge **15** may include a chamber or radius to facilitate entry into the female type second interface member **24** shown in **Figure 6(A)****.** **Figure 5(A)** also illustrates a circumferentially oriented protrusion **18** in the form of a single continuous rib on the first interface member **14.** **Figure 5(A)** further illustrates a male type channel communication section. This type of channel communication section is an exemplary (non-limiting) embodiment for connection to typical PEG tubes.

**Figure 5(B)** provides a sectional view designated as A-A for the embodiment shown in **5(A),** and as such shows the fluid flow path **42.**

**Figure 5(C)** provides an enlarged partial view designated as Detail B of the sectional view shown in **Figure 5(B). Figure 5(C)** illustrates a cross sectional view of the circumferentially oriented protrusion **18,** in this case embodied as a single continuous rib.

Referring now to **Figures 6(A)****-(C),** which provide assorted views of an embodiment of a second connector **22,** **Figure 6(A)** shows an elevated schematic of a second connector **22.** In this instance, the second interface member **24** is a female type connector with a first edge **25.** The first edge **25** has a chamber or radius to facilitate entry of the male type first interface member **14** shown in **Figure 5(A)****.**

**Figure 6(A)** further illustrates a male type channel communication section. This type of channel communication section is an exemplary (non-limiting) embodiment for connection to typical PEG tubes.

**Figure 6(B)** provides a sectional view designated as B-B for the embodiment shown in **6(A),** and as such shows the fluid flow path **42.**

**Figure 6(C)** provides an enlarged partial view designated as Detail A of the sectional view shown in **Figure 6(B). Figure 6(C)** illustrates a cross sectional view of the protrusion retention recess **28** as shown to accept the circumferentially oriented protrusion **18** of **Figure 5(A)****.** **Figure 6(C)** also illustrates a chamber on the first edge **25.**

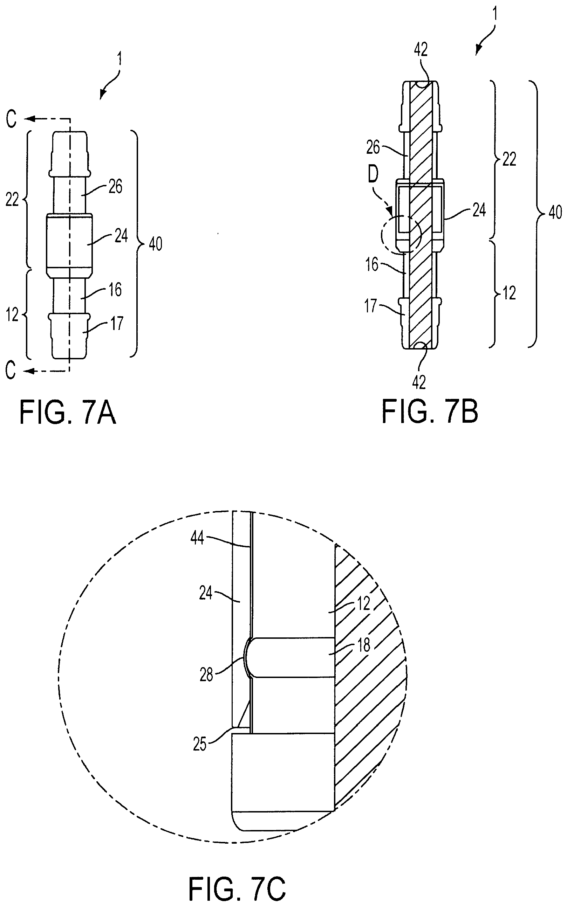

Referring now to **Figure 7(A), Figure 7(A)** provides and elevation view of an embodiment of the coupling **40** formed by the joining of the first connector **12** shown in **Figure 5(A)** and the second connector **22** show in **Figure 6(A)****.**

**Figure 7(B)** provides a sectional view designated as C-C for the embodiment shown in **Figure 7(A). Figure 7(B)** illustrates the embodiment of the coupling **40** formed by the joining of the first connector **12** shown in **Figure 5(A)** and the second connector **22** show in **Figure 6(A)****.** The fluid flow path **42** through the coupling is identified.

**Figure 7(C)** provides an enlarged partial view designated as Detail D of the sectional view shown in **Figure 7(B)****.** In this partial view it is shown how a cross section of the circumferentially oriented protrusion **18** and the protrusion retention recess **28** communicate while the first connector **12** and the second connector **22** are joined to form a coupling **40.** The faying surface **44** of the coupling is also shown.

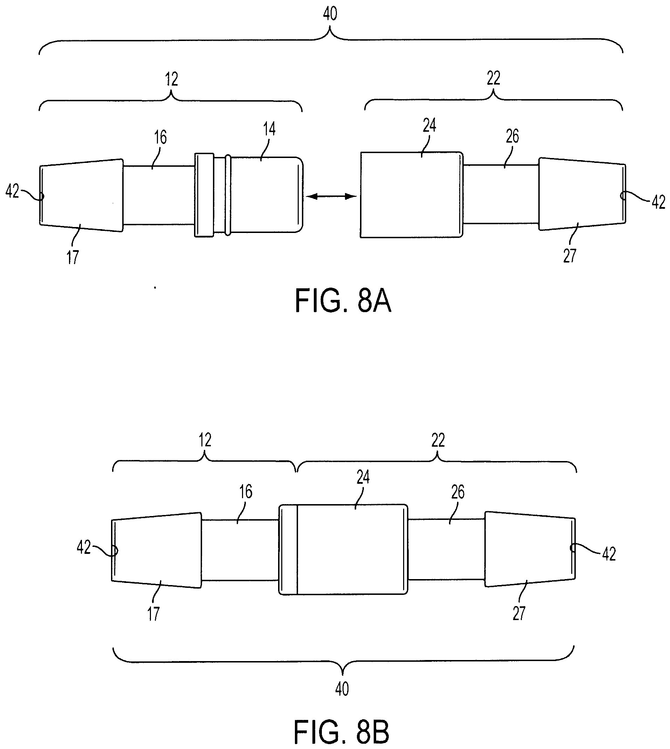

Referring now to **Figure 8, Figures 8(A)-8(B)** provide a perspective view of an embodiment of the coupling **40** in a disconnected position and connected position, respectively. **Figure 8(A)** provides a perspective view of an embodiment of the coupling **40** in a disconnected position, which includes a first connector **12** that is disconnected from a second connector **22.** As shown, the channel communication section **16** has a barb-like appendage **17** to facilitate communication with a channel (not shown). Similarly, the channel communication section **26** has a barb-like appendage **27** to facilitate with a channel (not shown). The first connector **12** and the second connector **22** are configured to provide a fluid flow path **42** there through. **Figure 8(B)** provides a perspective view of an embodiment of the coupling **40** of the coupling shown in **Figure 8(A)** whereby the first connector **12** is joined to the second connector **22** to form the coupling **40.** The coupling **40** is configured to provide for the fluid flow path **42** to extend through the coupling **40** to allow fluid to flow from the first connector **12** to the second connector **22.** As shown, the channel communication section **16** has a barb-like appendage **17** to facilitate communication with a channel (not shown). Similarly, the channel communication section **26** has a barb-like appendage **27** to facilitate communication with a channel (not shown).

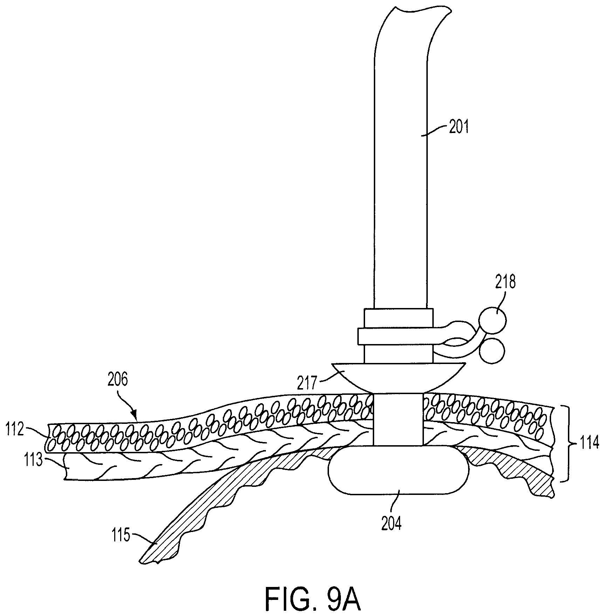

Referring now to **Figure 9(A), Figure 9(A)** provides an illustration of an elevation schematic view of a PEG tube **201.** The PEG tube **201** is shown traversing the abdominal wall **114** (cutanious wall **112** and subcutaneous wall **113**) and gastric wall **115** of the subject **206.** Also shown is an external bolster **217** with a twist lock **218** located proximal to the abdominal wall **114.** Also shown is an internal portion **204** inside the subject **206;** whereby in the instant illustration the internal portion is disclosed as a bumper.

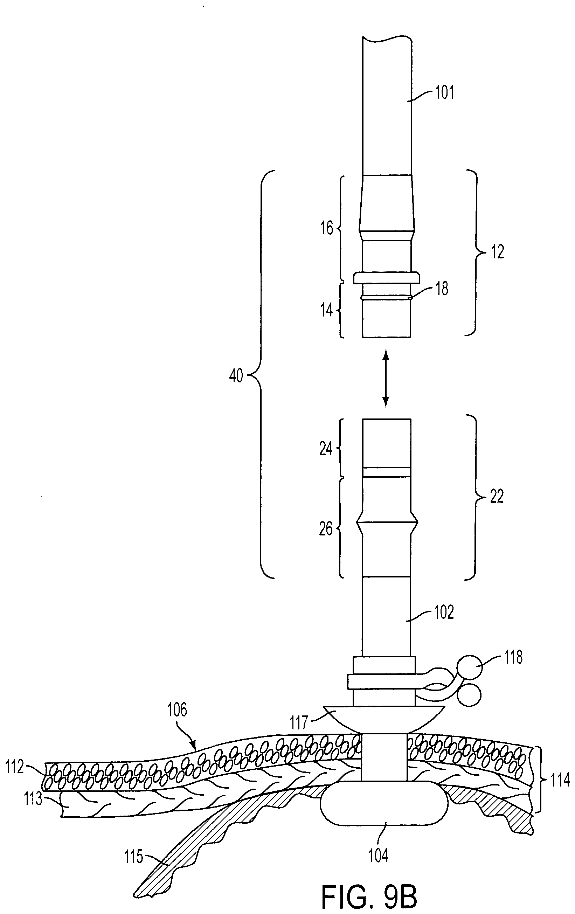

**Figures 9(B)****-(C)** provide an elevation schematic view of an embodiment of the coupling **40** in a disconnected position and connected position, respectively.

**Figure 9(B)** provides a perspective view of an embodiment of the coupling **40** in a disconnected position, which includes first connector **12** that is disconnected from the second connector **22.** **Figure 9(B)** provides an illustration of a PEG tube having a first channel **101** and second channel **102** with an embodiment of the coupling **40** installed therewith. Also shown is an external bolster **117** with a twist lock **118** located between the coupling **40** and the subject **106** proximal to the abdominal wall **114.** The coupling **40** is installed externally near to the abdominal wall **114** (or in a location as desired or required) of the subject **106** and is comprised of a first connector **12** communicating with a first channel **101** and a second connector **22** communicating with a second channel **102.** The second channel **102** passes through the subject's abdominal wall **114** and gastric wall **115** and has an internal portion **104** inside the subject **106;** whereby in the instant illustration the internal portion is disclosed as a bumper. The first connector **12** includes a first interface member **14** with a protrusion **18** to be to be matted with the second interface member **24** of the second connector **22.** It should be noted that the protrusion **18** of the first connector **12** may be mated with protrusion retention recess (not shown) of the second interface member **24.**

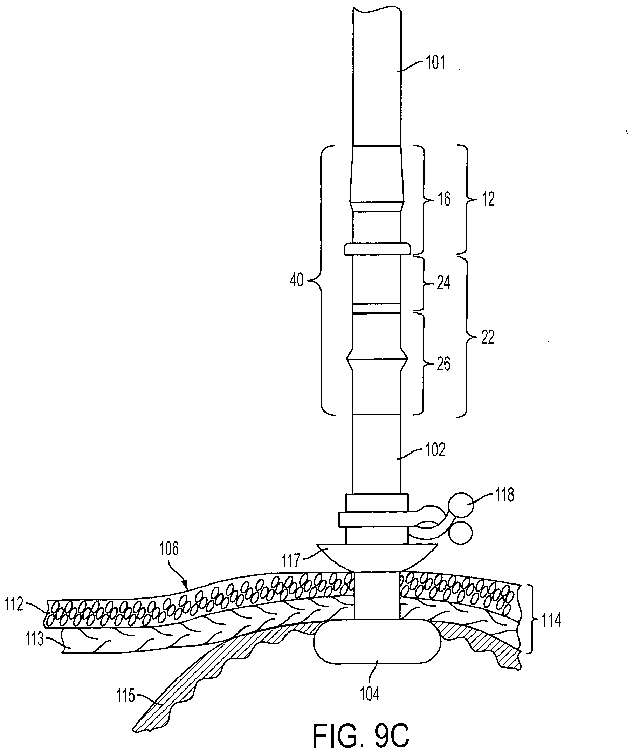

**Figure 9(C)** provides a perspective view of an embodiment of the coupling **40** shown in **Figure 9(B)** wherein the coupling **40** is in a connected position having the first connector **12** connected with the second connector **22.** It should be noted that first interface member **14** and its protrusion **18** is inside the second interface member **24** of the second connector **22** and therefore is not visible in the illustration.

Furthermore, it should be appreciated that various components of this device may be manufactured or formed from a multitude of materials that satisfy the working requirements of the invention. This includes, but is not limited to, plastics, polymers, composites, metals, alloys and any combination thereof. This also includes, but is not limited to, materials molded or otherwise formed in order to have changing properties in any fashion including, but not limited to, along their length or across their section. This change in properties may either be by section or continuous in nature. The materials selected for the device **1** may be selected based on ease of manufacturing, price, material properties such as density, strength, modulus of elasticity, electrical or thermal conductivity, and biological compatibility.

The device **1,** any of its components or sub-components, or any portions thereof may be manufactured or formed from a multitude of materials that satisfy the working requirements of the invention. This includes, but is not limited to, plastics, polymers, composites, metals, alloys and any combination thereof. This also includes, but is not limited to, materials molded or otherwise formed in order to have changing properties in any fashion including along their length or across their section. This change in properties may either be sectioned or continuous in nature. The materials selected for the device 1 may be selected based on ease of manufacturing, price, material properties such as density, strength, modulus of elasticity, electrical or thermal conductivity, and biological compatibility.

The applicant contemplates within the context of this invention that it may be produced in any geometrical form with variable length, width, shape, size, or other dimensional variability to match the requirements of specific applications for use.

It should be appreciated that the device **1** may be manufactured in a variety of ways. Specifically, this includes forming, molding, casting, forging, or otherwise producing components, sub-components, or portions thereof. The device may be produced as an assembly of parts wherein those parts are attached in any manner, including but not limited to fusing, welding, friction fits, threaded connections, snap connections, adhesives, or any other method for connecting one component, sub-component, or any portion thereof to another component, sub-component or portion thereof. The device **1** may also be manufactured so as to combine different functional elements into a single, multi-function component that would take on the function of two otherwise separate components.

It should be appreciated that as discussed herein, a subject may be a human or any animal. It should be appreciated that an animal may be a variety of any applicable type, including, but not limited thereto, mammal, veterinarian animal, livestock animal or pet type animal, etc. As an example, the animal may be a laboratory animal specifically selected to have certain characteristics similar to human (e.g. rat, dog, pig, monkey), etc. It should be appreciated that the subject may be any applicable human patient, for example.

It should be appreciated that various sizes, dimensions, contours, rigidity, shapes, flexibility and materials of any of the components or portions of components in the various embodiments discussed throughout may be varied and utilized as desired or required. Similarly, locations and alignments of the various components may vary as desired or required.

It should be appreciated that any of the components or modules referred to with regards to any of the present invention embodiments discussed herein, may be integrally or separately formed with one another. Further, redundant functions or structures of the components or modules may be implemented.

It should be appreciated that the device 1 and related components discussed herein may take on all shapes along the entire continual geometric spectrum of manipulation of x, y and z planes to provide and meet the anatomical and structural demands and operational requirements. Moreover, locations and alignments of the various components may vary as desired or required.

### EXAMPLES

Practice of an aspect of an embodiment (or embodiments) of the invention will be still more fully understood from the following examples and experimental results, which are presented herein for illustration only and should not be construed as limiting the invention in any way.

### Example and Experimental Set No. 1

### Data Acquisition Study:

The Applicants' UVA Institutional Review Board (IRB)-approved trial (UVA-IRB# 14526) was conducted on 60 patients to quantify the force required to *intentionally* remove a PEG from the stomach by gentle external traction. This data was used to determine the ideal force that an embodiment of the present invention device should disconnect. The force to remove the PEG was quantified by using a force measurement gauge (American Weigh® SR-20 Digital Hanging Scale 20 x 0.01kg, and Extech Instruments Digital Force Gauge: Model 475044) to remove the PEGs out intentionally in clinic by external traction. This data allowed determination of the force (i.e., decoupling force) at which the device should separate in order to prevent the PEG from accidentally being pulled through the abdominal wall.

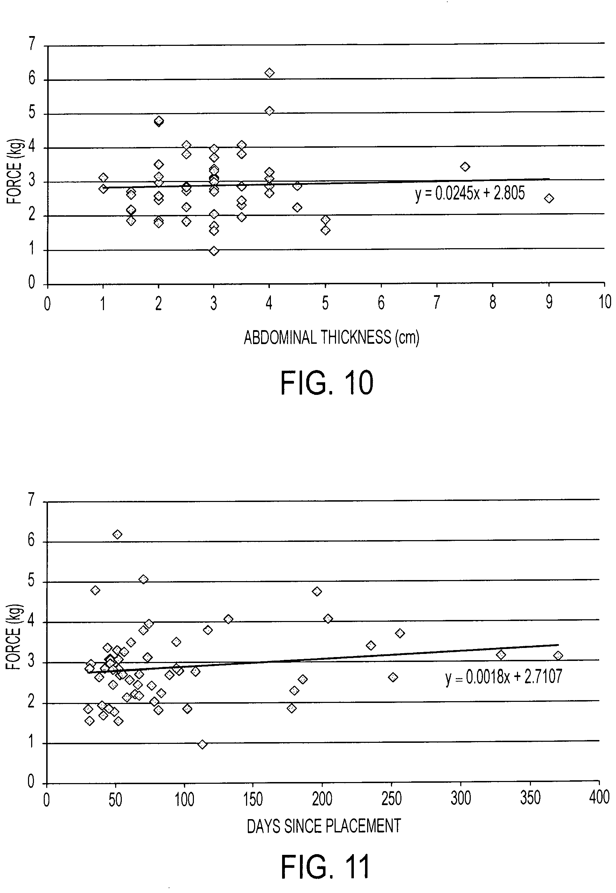

Referring to the graph of **Figure 10****,** data demonstrates the force to intentionally remove a PEG tube is not related to thickness of the abdominal wall. The trendline (y=0.0245x+2.805) is nearly horizontal (slope of zero) indicating no relationship between the abdominal thickness and force of dislodgement. Accordingly, as the device is removed when the internal bumper collapses to the diameter of the tube allowing easy passage through the abdominal wall, regardless of how far it must travel. This information reveals a single device, with a particular break force, will be applicable to all patients regardless of patient size or abdominal girth. The graph of **Figure 10** depicts the force (kg) of external traction for removal of PEG tubes through abdominal wall versus the thickness (cm) of the abdominal wall.

Referring to the graph of **Figure 11****,** the graph displays the days since placement or the "age" of the PEG tube. The data suggests the "age" of the tube is unrelated to the force required for removal (y=0.0018x + 2.7107). The graph illustrates the force of external traction (kg) versus "age" (days) of the PEG tube.

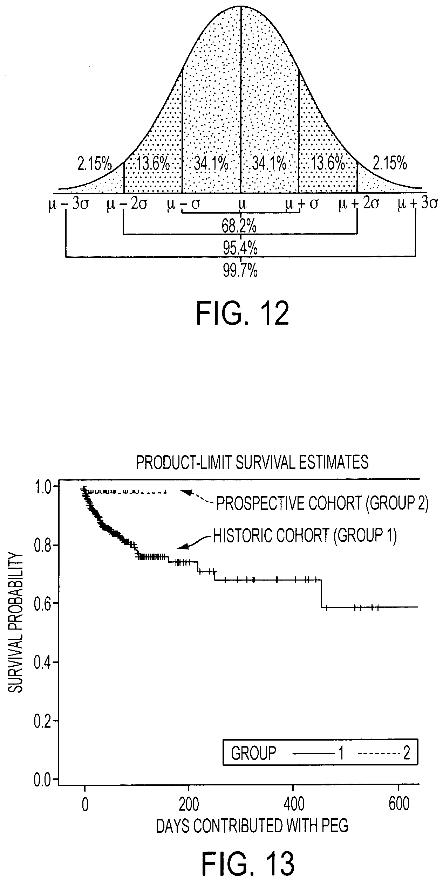

Turning to **Figure 12****,** the mean, ± the standard error of the mean, for the pull force from these 60 patients is 2.88 kg ± 0.12 with a standard deviation of 0.93 kg. The median pull force is 2.82 kg and the range is from 0.97 to 6.19 kg. Assuming a standard normal (Gaussian) distribution for this continuous data, one can determine the ideal break force. By taking two standard deviations above and below the mean one can predict to include 95.4% of all dislodgement data points. Accordingly, **Figure 12** graphically illustrates the Standard (Gaussian) distribution with mean (µl) and standard deviations (σ).

For example, two standard deviations (0.93 kg) below the mean (2.88 kg) is a value of 1.02 kg. This is the "force" at which the device will separate. If the device disconnects at approximately 1.0 kg of force one can predict it will prevent 97.6% of PEG dislodgements as calculated from the original data.

In summary, an aspect of an embodiment of the present invention PEG coupling device will prevent accidental removal of the PEG feeding tube by being a point of disconnection to prevent the PEG tube's internal bumper from collapsing and dislodging through the abdominal wall. External traction placed anywhere along the length of the tubing will cause the two-piece device to separate, relieving all pressure from the internal bumper preventing its collapse and dislodgement. This device is applicable for all PEGs with a soft internal bumper and not those with an intragastric balloon.

A great benefit of various embodiments of the present invention device is that it does not require any pre-market modification of the PEG tubes and insertion kits.

This can be added to the PEG tubing following placement and does not require a well-healed gastrocutaneous fistula tract (as is required for a replacement balloon gastrostomy tube). It is also designed to fit any 24-French PEG tubing and therefore is applicable regardless of PEG brand. Patient safety organizations have already become concerned with the restraining of patients, and more regulations have been put into place restricting the use of wrist restraints and hand mitts. With increasing regulations such as these, it is anticipated that there will be more frequent PEG dislodgements and an even greater need for a novel safety mechanism.

### Example and Experimental Set No. 2

An aspect of an embodiment of the present invention and related method is intended to prevent the accidental dislodgement of percutaneous endoscopic gastrostomy (PEG) tubes, as shown in **Figure 9(A)****.** It is intended to be a point of disconnection when force (or external traction) is placed anywhere along the length of the tubing external to the patient.

The standard PEG tube provides direct access to the stomach and provides enteral nutrition via the long, flexible feeding tube with a soft internal bumper. Conveniently, the soft inner bumper of the PEG collapses and slides out through the tube tract, allowing the PEG to be removed with relatively minimal external traction. This feature allows easy, intentional removal in an outpatient, clinic setting without the need for an operation or sedation when the patient has recovered from their initial insult.

Consequently, due to this flexible inner bumper, a major complication is premature removal or accidental dislodgement, from which significant morbidity and mortality may occur. Secondary complications occur with premature removal as the gastrocutaneous fistula tract has not fully matured allowing the stomach to separate from the anterior abdominal wall and the open gastrostomy to leak gastric contents. Complications include peritonitis requiring laparotomy, abdominal wall necrotizing fasciitis, candida peritonitis, and hemoperitoneum following gastrostomy tube reinsertion. Accidental dislodgement rates are well published and have been reported as up to 12.8% when followed longitudinally for the lifetime of the originally placed PEG. See Rosenberger et al., "Late accidental dislodgement of the percutaneous endoscopic gastrostomy: an underestimated burden on patients and the healthcare system".

While early dislodgements can be clinically devastating, late dislodgements after gastrocutanous fistula maturation are less detrimental, however, they may require expensive emergency department visits, surgical consultations, replacement tubes, and radiographic confirmation of position. See Rosenberger et al., "Late accidental dislodgement of the percutaneous endoscopic gastrostomy: an underestimated burden on patients and the healthcare system".

One aspect of an embodiment of the present invention is a solution to what has become the "Achilles Heel" of the PEG tube; accidental dislodgement.

Applicants conducted a study at the University of Virginia to assess the effectiveness of an embodiment of the present invention in a PEG tube as shown in **Figures 9(B)** and **9(C)****.** The primary endpoint was the number of accidental dislodgements of the principally placed PEG tube. This study was designed as a prospective longitudinal cohort for comparison against a well-defined historical cohort. See Rosenberger et al., "Late accidental dislodgement of the percutaneous endoscopic gastrostomy: an underestimated burden on patients and the healthcare system".

Following standard PEG placement, an embodiment of the invention was placed in close proximity to the patient's abdominal wall. Typically, PEG tubing was cut transversely approximately 2 cm above the top of the external bumper and the device was installed. This resulted in relatively short first channel and a longer second channel connected to the feeding apparatus.

A total of 53 patients completed the initial phase of the study, 1 resulting in accidental PEG dislodgement, 37 in intentional removal of the PEG, 0 with exchange of the original PEG for a replacement balloon gastrostomy, and 15 in death with the device in place. A Kaplan-Meier survival analysis was performed comparing the prospective cohort (PEG tubes with an embodiment of the present invention, N=53) with the historic cohort (PEG tubes without an embodiment of the invention, N=563). The analysis shows a clear and significant longer survival of a single PEG tube with an embodiment of the invention in place. The exemplary embodiment of invention is shown to prevent accidental dislodgement and allow any given PEG to remain in place longer than previously shown. Referring to the graph of **Figure 13****,** the log-rank test reveals a significant difference (p=0.043). The graph of **Figure 13** reveals a Kaplan-Meier analysis for current prospective cohort (Group 2) versus historic cohort (Group 1).
The top line represents the prospective cohort with invention in place and the bottom line represents the historical cohort without invention in place

### Example and Experimental Set No. 3

It should be appreciated that as an embodiment of the invention has demonstrated effectiveness in preventing the accidental dislodgment of PEG tubes, further embodiments should be equally able to prevent accidental dislodgment of other tubes and channels that have an internal portion inside a subject. Such other embodiments of tubes or channels, and applications include, but are in no way limited to:
Foley Catheter - where the internal portion of the catheter is positioned in the bladder for gravity drainage. An external force on the tubing can cause disruption of the internal portion of the catheter. An embodiment of the invention could be installed in-line, a short distance from the body and would separate when a tractive force was applied that would typically disrupt the internal portion of the catheter.

Intraventricular drain - where the internal portion of the drain channel is positioned inside the ventricles of the brain and where an external force on the external portion of the channel can cause disruption of the internal portion of the channel. An embodiment of the invention could be installed in-line, a short distance from the body and would separate when a tractive force was applied that would typically disrupt the internal portion of the channel.

Chest tube - where the internal portion of the chest tube sits inside the thoracic cavity between the lungs and the chest wall. The chest tube is designed to drain air, fluid, or blood from the thoracic cavity and external force on the tube may cause disruption of the internal position of the tube. An embodiment of the invention could be installed in-line, a short distance from the body and would separate when a tractive force was applied that would typically disrupt the internal portion of the tube.

Nephrostomy tubes - where an internal "pigtail end" of the catheter sits in the renal pelvis to drain urine from the kidney. This pigtail end may be disrupted when an external force is applied to the external portion of the catheter. An embodiment of the invention could be installed in-line, a short distance from the body and would separate when a tractive force was applied that would typically disrupt the pigtail end of the catheter.

Percutaneous transhepatic cholangiography ("PTC") tube - where an internal channel is placed into the biliary tree to allow bile drainage. Such channel can be disrupted when a force is applied to the external portion of the channel. An embodiment of the invention could be installed in-line, a short distance from the body and would separate when a tractive force was applied that would typically disrupt the internal portion of the channel.

Pigtail catheters for abscess drainage - where an internal "pigtail end" of the catheter sits in any number of intra-abdominal locations to drain fluid from an abscess. This pigtail end may be disrupted when an external force is applied to the external portion of the catheter. An embodiment of the invention could be installed in-line, a short distance from the body and would separate when a tractive force was applied that would typically disrupt the pigtail end of the catheter.

Generally, any channel that has an internal portion inside a subject is subject to disruption when a force is applied to the external portion of the channel. An embodiment of this invention may be placed in-line with the channel to prevent the disruption of the internal portion of the channel when an applicable force is applied beyond the invented device. Similarly, an embodiment of the invention may be implemented with an optimal or desired decoupling force according to the teachings, techniques, structures, components and principles set forth in this disclosure regarding the various embodiments or aspects of the present invention.

### Additional Examples

Example 1. An assembly device for use in a medical environment to be used with a first channel and a second channel, wherein the first channel has an internal portion located at an intended position inside a subject. The device may comprise: a first connector comprising a first interface member and a channel communication section; a second connector comprising a second interface member and a channel communication section; the first interface member of the first connector and the second interface member of the second connector configured to join together to form a coupling configured to allow fluid to flow between the first channel and the second channel, the coupling having a decoupling force; wherein the coupling is joined to the first channel and the second channel at the communication section of the first connector and the communication section of the second connector; and wherein the decoupling force is the force required to separate the first connector and the second connector from one another to allow the internal portion to maintain its intended position in the subject.
Example 2. The device of example 1, wherein the first connector comprises a male connector and the first interface member comprises an insert section.
Example 3. The device of example 2, wherein the second connector comprises a female connector and the second interface member comprises a receiving section, whereby the coupling is provided by the receiving section receiving the insert section to provide the joining together.
Example 4. The device of example 1 (as well as subject matter of one or more of any combination of examples 2-3), wherein the first channel and/or second channel comprises: a tube, a conduit, a port or any combination thereof.
Example 5. The device of example 4 (as well as subject matter of one or more of any combination of examples 2-3), wherein the tube comprises a percutaneous endoscopic gastrostomy (PEG).
Example 6. The device of example 1 (as well as subject matter of one or more of any combination of examples 2-5), wherein the decoupling force is between 0.5 kilogram-force and 2.5 kilograms-force.
Example 7. The device of example 1 (as well as subject matter of one or more of any combination of examples 2-6), wherein the decoupling force is between 1.1 kilograms-force and 1.3 kilograms-force.
Example 8. The device of example 1 (as well as subject matter of one or more of any combination of examples 2-7), wherein the channel communication section 16 on the first connector is configured to have an attachment means for attaching to the first channel and/or the second channel.
Example 9. The device of example 1 (as well as subject matter of one or more of any combination of examples 2-8), wherein the channel communication section 26 on the second connector is configured to have an attachment means for attaching to the first channel and/or the second channel.
Example 10. The assembly of example 1 (as well as subject matter of one or more of any combination of examples 2-9), wherein the joining of the first interface member of the first connector and the second interface member of the second connector provides faying surfaces, wherein the faying surfaces forms a leak inhibiting seal.
Example 11. The device of example 1 (as well as subject matter of one or more of any combination of examples 2-10), wherein the first interface member of the first connector includes one or more circumferentially oriented protrusions.
Example 12. The device of example 7 (as well as subject matter of one or more of any combination of examples 1-6 or 8-11), wherein the one or more circumferentially oriented protrusions are a continuous segment.
Example 13. The device of example 7 (as well as subject matter of one or more of any combination of examples 1-6 or 8-12), where the one or more circumferentially oriented protrusions are discontinuous segments.
Example 14. The device of example 7 (as well as subject matter of one or more of any combination of examples 1-6 or 8-13), wherein the second interface member of second connector includes one or more protrusion retention recesses.
Example 15. The device of example 1 (as well as subject matter of one or more of any combination of examples 2-14), wherein the decoupling force is the force required to separate the first connector and the second connector from one another to allow the internal portion to maintain its intended position in the subject and to allow the separated the first connector and the second connector to remain joined to their the respective channels.
Example 16. An assembly device for use in a medical environment to be used with a first channel and a second channel, wherein the first channel has an internal portion located at an intended position inside a subject. The device may comprise: a first connector ; a second connector; the first connector and the second connector configured to join together to form a coupling configured to allow fluid to flow between the first channel and the second channel, the coupling having a decoupling force; wherein the coupling is joined between the first channel and the second channel; and wherein the decoupling force is the force required to separate the first connector and the second connector from one another to allow the internal portion to maintain its intended position in the subject. Moreover, the system may include subject matter of one or more of any combination of examples 1-15.
Example 17. The device of example 16 (as well as subject matter of one or more of any combination of examples 1-15), wherein the first channel and/or second channel comprises: a tube, a conduit, a port or any combination thereof.
Example 18. The device of example 17 (as well as subject matter of one or more of any combination of examples 1-15), wherein the tube comprises a percutaneous endoscopic gastrostomy (PEG).
Example 19. The device of example 16 (as well as subject matter of one or more of any combination of examples 1-15 or 17-18), wherein the decoupling force is between 0.5 kilogram-force and 2.5 kilograms-force.
Example 20. The device of example 16 (as well as subject matter of one or more of any combination of examples 1-15 or 17-19), wherein the decoupling force is between 1.1 kilograms-force and 1.3 kilograms-force.
Example 21. The device of example 16 (as well as subject matter of one or more of any combination of examples 1-15 or 17-20), wherein the first connector is attached to the first channel using an attachment means.
Example 22. The device of example 16 (as well as subject matter of one or more of any combination of examples 1-15 or 17-21), wherein the first connector is attached to the first channel by way of at least one of the following connectors: male to female friction connector, magnetic connector, connector that uses adhesive, friction connection, ridge/valley connection, snap/click connection, O-ring connection, and screw/twist type mechanism connection.
Example 23. The device of example 16 (as well as subject matter of one or more of any combination of examples 1-15 or 17-22), wherein the second connector is attached to the second channel using an attachment means.
Example 24. The device of example 16 (as well as subject matter of one or more of any combination of examples 1-15 or 17-23), wherein the second connector is attached to the second channel by way of at least one of the following connectors: male to female friction connector, magnetic connector, connector that uses adhesive, friction connection, ridge/valley connection, snap/click connection, O-ring connection, and screw/twist type mechanism connection.
Example 25. The device of example 16 (as well as subject matter of one or more of any combination of examples 1-15 or 17-24), wherein the first connector is joined to the second connector forming the coupling, wherein the coupling comprises at least one of the following: male to female friction connector, magnetic connector, connector that use adhesive, friction connection, ridge/valley connection, snap/click connection, O-ring connection, and screw/twist type mechanisms connection.
Example 26. The device of example 16 (as well as subject matter of one or more of any combination of examples 1-15 or 17-25), wherein the decoupling force is the force required to separate the first connector and the second connector from one another to allow the internal portion to maintain its intended position in the subject and to allow the first connector to remain joined to the first channel and the second connector to remain connected to the second channel.
Example 27. A system for use in a medical environment, wherein the system comprises: a first channel; a second channel, wherein the first channel has an internal portion located at an intended position inside a subject; and an assembly device. The assembly device may comprise: a first connector comprising a first interface member and a channel communication section; a second connector comprising a second interface member and a channel communication section; the first interface member of the first connector and the second interface member of the second connector configured to join together to form a coupling configured to allow fluid to flow between the first channel and the second channel, the coupling having a decoupling force; wherein the coupling is joined to the first channel and the second channel at the communication section of the first connector and the communication section of the second connector; and wherein the decoupling force is the force required to separate the first connector and the second connector from one another to allow the internal portion to maintain its intended position in the subject. Moreover, the system may include subject matter of one or more of any combination of examples 1-26.
Example 28. A system for use in a medical environment, wherein the system comprises: a first channel; a second channel, wherein the first channel has an internal portion located at an intended position inside a subject; and an assembly device. The assembly device may comprise: a first connector; a second connector; the first connector and the second connector configured to join together to form a coupling configured to allow fluid to flow between the first channel and the second channel, the coupling having a decoupling force; wherein the coupling is joined between the first channel and the second channel; and wherein the decoupling force is the force required to separate the first connector and the second connector from one another to allow the internal portion to maintain its intended position in the subject. Moreover, the system may include subject matter of one or more of any combination of examples 1-27.
Example 29. A method used in a medical environment to be used with a first channel and a second channel, wherein the first channel has an internal portion located at an intended position inside the subject. The method may comprise: providing a first connector; providing a second connector; joining the first connector and the second connector to form a coupling to allow fluid to flow between the first channel and the second channel, the coupling having a decoupling force; wherein the coupling is joined to the first channel and the second channel; and wherein the decoupling force is the force required to separate the first connector and the second connector from one another to allow the internal portion to maintain its intended position. Moreover, the system may include subject matter of one or more of any combination of examples 1-28.
Example 30. The device of example 29 (as well as subject matter of one or more of any combination of examples 1-28), wherein the first connector comprises a male connector and the first interface member comprises an insert section.
Example 31. The device of example 30 (as well as subject matter of one or more of any combination of examples 1-28), wherein the second connector comprises a female connector and the second interface member comprises a receiving section, whereby the coupling is provided by the receiving section receiving the insert section to provide the joining together.
Example 32. The method of example 29 (as well as subject matter of one or more of any combination of examples 1-28 or 30-31), wherein the first channel and/or second channel comprises: a tube, a conduit, a port or any combination thereof.
Example 33. The method of example 32 (as well as subject matter of one or more of any combination of examples 1-28 or 30-31), wherein the tube comprises a percutaneous endoscopic gastrostomy (PEG).
Example 34. The method of example 29 (as well as subject matter of one or more of any combination of examples 1-28 or 30-33), wherein the decoupling force is between 0.5 kilogram-force and 2.5 kilograms-force.
Example 35. The method of example 29 (as well as subject matter of one or more of any combination of examples 1-28 or 30-34), wherein the decoupling force is between 1.1 kilograms-force and 1.3 kilograms-force.
Example 36. The method of example 29 (as well as subject matter of one or more of any combination of examples 1-28 or 30-35), wherein joining of the first interface member of the first connector and the second interface member of the second connector provides faying surfaces, wherein the faying surfaces forms a leak inhibiting seal.
Example 37. The method of example 29 (as well as subject matter of one or more of any combination of examples 1-28 or 30-36), wherein the decoupling force is the force required to separate the first connector and the second connector from one another to allow the internal portion to maintain its intended position in the subject and to allow the first connector to remain joined to the first channel and the second connector to remain connected to the second channel.
Example 38. A method used in a medical environment, whereby the method comprises: providing a first channel; providing a second channel, wherein the first channel has an internal portion located at an intended position inside the subject; providing a first connector; providing a second connector; joining the first connector and the second connector to form a coupling to allow fluid to flow between the first channel and the second channel, the coupling having a decoupling force; wherein the coupling is joined to the first channel and the second channel; and wherein the decoupling force is the force required to separate the first connector and the second connector from one another to allow the internal portion to maintain its intended position. Moreover, the system may include subject matter of one or more of any combination of examples 1-37.
Example 39. A method of manufacturing any of the devices or systems (or portions thereof) provided in one or more of any combination of examples 1-28; by implementing, but not limited thereto, fabrication techniques and material selection known to one skilled in art.

## Claims

1. An assembly device for use in the percutaneous endoscopic gastrostomy to be used with a first channel (101) and a second channel (102), wherein the first channel (101) has an internal portion (104) located at an intended position inside a human body, said device comprising:
a first connector (12) comprising a first interface member (14) and a channel communication section (16), where at least a portion of said first channel communication section (16) is provided inside said first channel (101);
**characterized in that**
a second connector (22) comprising a second interface member (24) and a channel communication section, where at least a portion of said second channel communication section (26) is provided inside said second channel (102);
said first interface member (14) of said first connector (12) and said second interface member (24) of said second connector (22) configured to join together to form a coupling configured to allow fluid to flow between the first channel (101) and the second channel (102), said coupling having a predetermined decoupling force in the axial direction of the coupling;
wherein said coupling is joined to the first channel (101) and the second channel (102) at said communication section (16) of said first connector (12) and said communication section (26) of said second connector (22); wherein said predetermined axial decoupling force is the force required to separate said first connector (12) and the second connector (22) from one another along with the internal portion (104) of the first channel (101) maintaining its intended position in the human body when the second channel (102) is pulled away from the human body; and
wherein the decoupling force is between 1.1 kilograms-force and 1.3 kilograms-force, i.e. between 10.8 N and 12.7N.

2. The device of claim 1, wherein said first connector (12) comprises a male connector and said first interface member (14) comprises an insert section, wherein said second connector (22) comprises a female connector and said second interface member (24) comprises a receiving section, whereby the coupling is provided by said receiving section receiving said insert section to provide the joining together.

3. The device of claim 1, wherein said first channel (101) and/or second channel (102) comprises: a tube, a conduit, a port or any combination thereof, wherein said tube comprises a percutaneous endoscopic gastrostomy (PEG).

4. The device of claim 1, wherein said channel communication section (16) on said first connector (12) is configured to have an attachment means for attaching to the first channel (101) and/or the second channel (102).

5. The device of claim 1, wherein said channel communication section (26) on said second connector (22) is configured to have an attachment means for attaching to the first channel (101) and/or the second channel (102).

6. The assembly of claim 1, wherein said joining of said first interface member (14) of said first connector (12) and said second interface member (24) of said second connector (22) provides faying surfaces, wherein said faying surfaces forms a leak inhibiting seal.

7. The device of claim 1, wherein said first interface member (14) of said first connector (12) includes one or more circumferentially oriented protrusions.

8. The device of claim 7, wherein said one or more circumferentially oriented protrusions are a continuous segment.

9. The device of claim 7, where said one or more circumferentially oriented protrusions are discontinuous segments.

10. The device of claim 7, wherein said second interface member (24) of second connector (22) includes one or more protrusion retention recesses.

11. The device of claim 1, wherein said decoupling force is the force required to separate said first connector (12) and said second connector (22) from one another to allow the internal portion (104) to maintain its intended position in the human body and to allow the separated said first connector (12) and said second connector (22) to remain joined to their said respective channels.

12. The device of claim 1, wherein said first connector (12) is joined to said second connector (22) forming said coupling, wherein said coupling comprises at least one of the following: male to female friction connector, magnetic connector, connector that use adhesive, friction connection, ridge/valley connection, snap/click connection, O-ring connection, and screw/twist type mechanisms connection.

13. A system for use in the percutaneous endoscopic gastrostomy, wherein said system comprises:
a first channel (101);
a second channel (102), wherein the first channel (101) has an internal portion (104) located at an intended position inside a human body; and
an assembly device according to claim 1.

## Patentansprüche

1. Montagevorrichtung zur Verwendung bei der perkutanen endoskopischen Gastrostomie, für die Verwendung mit einem ersten Kanal (101) und einem zweiten Kanal (102), wobei der erste Kanal (101) einen Innenabschnitt (104) aufweist, der sich an einer vorgesehenen Position innerhalb eines menschlichen Körpers befindet, wobei die Vorrichtung umfasst:
ein erstes Verbindungsteil (12), das ein erstes Schnittstellenelement (14) und einen Kanalverbindungsabschnitt (16) umfasst, wobei mindestens ein Abschnitt des ersten Kanalverbindungsabschnitts (16) innerhalb des ersten Kanals (101) vorgesehen ist;
**dadurch gekennzeichnet, dass**
ein zweites Verbindungsteil (22), das ein zweites Schnittstellenelement (24) und einen Kanalverbindungsabschnitt (26) umfasst, wobei mindestens ein Abschnitt des zweiten Kanalverbindungsabschnitts (16) innerhalb des zweiten Kanals (102) vorgesehen ist;
wobei das erste Schnittstellenelement (14) des ersten Verbindungsteils (12) und das zweite Schnittstellenelement (24) des zweiten Verbindungsteils (22) zum Zusammenfügen konfiguriert sind, um eine Kupplung zu bilden, die konfiguriert ist, um zu ermöglichen, dass Fluid zwischen dem ersten Kanal (101) und dem zweiten Kanal (102) strömt, wobei die Kupplung eine vorbestimmte Entkopplungskraft in der axialen Richtung der Kupplung aufweist;
wobei die Kupplung mit dem ersten Kanal (101) und dem zweiten Kanal (102) an dem Verbindungsabschnitt (16) des ersten Verbindungsteils (12) und dem Verbindungsabschnitt (26) des zweiten Verbindungsteils (22) verbunden ist; wobei die vorgegebene axiale Entkopplungskraft die Kraft ist, die erforderlich ist, um das erste Verbindungsteil (12) und das zweite Verbindungsteil (22) voneinander zu trennen, zusammen mit dem Innenabschnitt (104) des ersten Kanals (101), der seine vorgesehene Position im menschlichen Körper beibehält, wenn der zweite Kanal (102) aus dem menschlichen Körper herausgezogen wird; und
wobei die Entkopplungskraft zwischen 1,1 Kilopond und 1,3 Kilopond, d. h., zwischen 10,8 N und 12,7 N liegt.

2. Vorrichtung nach Anspruch 1, wobei das erste Verbindungsteil (12) ein männliches Verbindungsteil ist und das erste Schnittstellenelement (14) einen Einführabschnitt umfasst, wobei das zweite Verbindungsteil (22) ein weibliches Verbindungsteil ist und das zweite Schnittstellenelement (24) einen Aufnahmeabschnitt umfasst, wobei die Kupplung durch den Einführabschnitt aufnehmenden Aufnahmeabschnitt bereitgestellt wird, um die Verbindung gemeinsam herzustellen.

3. Vorrichtung nach Anspruch 1, wobei der erste Kanal (101) und/oder der zweite Kanal (102) umfassen: ein Rohr, eine Leitung, einen Port oder eine beliebige Kombination davon, wobei das Rohr eine perkutane endoskopische Gastrostomie (PEG) umfasst.

4. Vorrichtung nach Anspruch 1, wobei der Kanalverbindungsabschnitt (16) an dem ersten Verbindungsteil (12) konfiguriert ist, ein Befestigungsmittel zum Befestigen an dem ersten Kanal (101) und/oder dem zweiten Kanal (102) aufzuweisen.

5. Vorrichtung nach Anspruch 1, wobei der Kanalverbindungsabschnitt (26) an dem zweiten Verbindungsteil (22) konfiguriert ist, ein Befestigungsmittel zum Befestigen an dem ersten Kanal (101) und/oder dem zweiten Kanal (102) aufzuweisen.

6. Anordnung nach Anspruch 1, wobei die Verbindung des ersten Schnittstellenelements (14) des ersten Verbindungsteils (12) und des zweiten Schnittstellenelements (24) des zweiten Verbindungsteils (22) Passflächen bereitstellt, wobei die Passflächen eine auslaufhemmende Dichtung bilden.

7. Vorrichtung nach Anspruch 1, wobei das erste Schnittstellenelement (14) des ersten Verbindungsteils (12) einen oder mehrere in Umfangsrichtung orientierte Vorsprünge umfasst.

8. Vorrichtung nach Anspruch 7, wobei der eine oder die mehreren in Umfangsrichtung orientierten Vorsprünge ein kontinuierliches Segment sind.

9. Vorrichtung nach Anspruch 7, wobei der eine oder die mehreren in Umfangsrichtung orientierten Vorsprünge ein nicht kontinuierliches Segment sind.

10. Vorrichtung nach Anspruch 7, wobei das zweite Schnittstellenelement (24) des zweiten Verbindungsteils (22) einen oder mehrere in Umfangsrichtung orientierte Vorsprungs-Rückhalteaussparungen umfasst.

11. Vorrichtung nach Anspruch 1, wobei die Entkopplungskraft die Kraft ist, die erforderlich ist, um das erste Verbindungsteil (12) und das zweite Verbindungsteil (22) voneinander zu trennen, um es dem Innenabschnitt (104) zu ermöglichen, seine vorgesehene Position in dem menschlichen Körper beizubehalten und es dem getrennten ersten Verbindungsteil (12) und dem zweiten Verbindungsteil (22) zu ermöglichen, mit ihren jeweiligen Kanälen verbunden zu bleiben.

12. Vorrichtung nach Anspruch 1, wobei das erste Verbindungsteil (12) mit dem zweiten Verbindungsteil (22) verbunden ist, was die Kupplung bildet, wobei die Kupplung mindestens eines der folgenden umfasst: männlich-zu-weiblich-Reibverbindungsteil, Magnetverbindungsteil, Verbindungsteil, das Klebstoff verwendet, Reibungsverbindung, Rippen-/Mulden-Verbindung, Schnapp-/Klick-Verbindung, O-Ring-Verbindung und Schraubtyp/Drehtyp-Mechanismusverbindung.

13. System zur Verwendung bei der perkutanen endoskopischen Gastrostomie, wobei das System umfasst:
einen ersten Kanal (101),
einen zweiten Kanal (102), wobei der erste Kanal (101) einen Innenabschnitt (104) aufweist, der an einer vorgesehenen Position innerhalb eines menschlichen Körpers angeordnet ist; und
eine Montagevorrichtung nach Anspruch 1.

## Revendications

1. Dispositif d'assemblage destiné à être utilisé pour une gastrostomie endoscopique percutanée à utiliser avec un premier canal (101) et un second canal (102), dans lequel le premier canal (101) possède une partie interne (104) située à un emplacement prévu à l'intérieur d'un corps humain, ledit dispositif comprenant :
un premier connecteur (12) comprenant un premier élément d'interface (14) et une section de communication de canal (16), dans lequel au moins une partie de ladite première section de communication de canal (16) est prévue à l'intérieur dudit premier canal (101) ;
**caractérisé en ce qu'**il comprend
un second connecteur (22) comprenant un second élément d'interface (24) et une section de communication de canal, dans lequel au moins une partie de ladite seconde section de communication de canal (26) est prévue à l'intérieur dudit second canal (102) ;
ledit premier élément d'interface (14) dudit premier connecteur (12) et ledit second élément d'interface (24) dudit second connecteur (22) sont configurés pour se joindre afin de former un couplage configuré pour permettre à un fluide de circuler entre le premier canal (101) et le second canal (102), ledit couplage ayant une force de découplage prédéterminée dans la direction axiale du couplage ;
dans lequel ledit couplage est joint au premier canal (101) et au second canal (102) au niveau de ladite section de communication (16) dudit premier connecteur (12) et de ladite section de communication (26) dudit second connecteur (22) ;
dans lequel ladite force de découplage prédéterminée est la force nécessaire pour séparer ledit premier connecteur (12) et le second connecteur (22) l'un de l'autre avec la partie interne (104) du premier canal (101) maintenant sa position prévue dans le corps humain lorsque le second canal (102) est sorti du corps humain ; et
dans lequel la force de découplage est comprise entre 1,1 kilogramme-force et 1,3 kilogramme-force, c'est-à-dire entre 10,8 N et 12,7 N.

2. Dispositif selon la revendication 1, dans lequel ledit premier connecteur (12) comprend un connecteur mâle et ledit premier élément d'interface (14) comprend une section d'insert, dans lequel ledit second connecteur (22) comprend un connecteur femelle et ledit second élément d'interface (24) comprend une section de réception, moyennant quoi le couplage est assuré par ladite section de réception qui reçoit ladite section d'insert afin d'assurer la jonction.

3. Dispositif selon la revendication 1, dans lequel ledit premier canal (101) et/ou ledit second canal (102) comprend : un tube, un conduit, un port ou n'importe quelle combinaison de ceux-ci, dans lequel ledit tube comprend une gastrostomie endoscopique percutanée (PEG).

4. Dispositif selon la revendication 1, dans lequel ladite section de communication de canal (16) sur ledit premier connecteur (12) est configurée pour avoir un moyen de fixation destiné à être relié au premier canal (101) et/ou au second canal (102).

5. Dispositif selon la revendication 1, dans lequel ladite section de communication de canal (26) sur ledit second connecteur (22) est configurée pour avoir un moyen de fixation destiné à être relié au premier canal (101) et/ou au second canal (102).

6. Ensemble selon la revendication 1, dans lequel ladite jonction dudit premier élément d'interface (14) dudit premier connecteur (12) et dudit second élément d'interface (24) dudit second connecteur (22) offre des surfaces de contact, dans lequel lesdites surfaces de contact forment un joint anti-fuites.

7. Dispositif selon la revendication 1, dans lequel ledit premier élément d'interface (14) dudit premier connecteur (12) comprend une ou plusieurs saillies orientées de manière circonférentielle.

8. Dispositif selon la revendication 7, dans lequel ladite ou lesdites saillies orientées de manière circonférentielle sont un segment continu.

9. Dispositif selon la revendication 7, dans lequel ladite ou lesdites saillies orientées de manière circonférentielle sont des segments discontinus.

10. Dispositif selon la revendication 7, dans lequel ledit second élément d'interface (24) du second connecteur (22) comprend un ou plusieurs renfoncements de retenue de saillies.

11. Dispositif selon la revendication 1, dans lequel ladite force de découplage est la force nécessaire pour séparer ledit premier connecteur (12) et ledit second connecteur (22) l'un de l'autre afin de permettre à la partie interne (104) de maintenir sa position prévue dans le corps humain et de permettre audit premier connecteur (12) et audit second connecteur (22) séparés de rester joints à leurs dits canaux respectifs.

12. Dispositif selon la revendication 1, dans lequel ledit premier connecteur (12) est joint audit second connecteur (22) en formant ledit couplage, dans lequel ledit couplage comprend au moins l'un de ce qui suit : un connecteur à friction mâle/femelle, un connecteur magnétique, un connecteur qui utilise un adhésif, une liaison à friction, une liaison à creux/vallées, une liaison à fermoir/à cliquet, une liaison à joint torique, et une liaison à mécanisme à vis/à torsion.

13. Système destiné à être utilisé pour une gastrostomie endoscopique percutanée, dans lequel ledit système comprend :
un premier canal (101) ;
un second canal (102), dans lequel le premier canal (101) possède une partie interne (104) située à un emplacement prévu à l'intérieur d'un corps humain ; et
un dispositif d'assemblage selon la revendication 1.
